# EUROPEAN PATENT APPLICATION

(11) **EP 3 566 724 A1**
(43) Date of publication of application: **13.11.2019**
(21) Application number: 19165283.3
(22) Date of filing: 26.03.2019
(51) Int. Cl.: A61L 15/44, A61L 15/60, B01J 20/28

(54) **METHOD OF MANUFACTURING ANTIBACTERIAL SUPERABSORBENT POLYMER ABSORBER CONTAINING METAL NANOPARTICLES**

(30) Priority: 09.05.2018 KR 20180053049
(71) Applicant: GeumVit Corp., Gyeonggi-do 14402 (KR)
(72) Inventor: KOH, Seok Keon, Gangnam-gu, Seoul 06255 (KR); OH, You Jin, Gwangju-si, Gyeonggi-do 12735 (KR); KIM, Hyung Deok, Goyang-si, Gueonggi-do 10522 (KR)
(74) Representative: Klunker IP Patentanwälte PartG mbB

(57) **Abstract**

The present invention relates to a method of manufacturing a superabsorbent polymer absorber containing metal nanoparticles and an application of the superabsorbent polymer absorber which is manufactured by the same and has antibacterial, sterilization, far infrared ray emission, prevention of blood oxidation, and mineral releasing functions. Specifically, the present invention relates to a technique in which metal nanoparticles such as copper, brass, bronze, silver, gold, germanium, and zinc are formed on the superabsorbent polymer by using a water-free manufacturing method, then, when the superabsorbent polymer absorbs water, the metal nanoparticles are naturally and uniformly dispersed, thereby allowing the superabsorbent polymer to exhibit inherent properties of the metal nanoparticles, and an application thereof.

## Description

### BACKGROUND OF THE INVENTION

### 1. Field of the Invention

The present invention relates to a method of manufacturing an antibacterial superabsorbent polymer absorber containing metal nanoparticles, and a use of the antibacterial superabsorbent polymer absorber prepared therefrom.

More specifically, the present invention relates to a method of directly preparing metal nanoparticles on a superabsorbent polymer in a water-free process, and using the prepared superabsorbent polymer as a liquid absorbent for various applications. In this case, when the superabsorbent polymer absorbs water, the prepared metal nanoparticles are uniformly dispersed into and out of the superabsorbent polymer at the same time, thereby imparting an antibacterial or sterilizing power to the liquid including the absorbed water. That is, when using in a product, the superabsorbent polymer absorber of the present invention may provide an antibacterial, sterilizing, or reducing power, as well as, a far infrared ray emission function, or mineral releasing function while maintaining the water absorbing potential of the polymer, thereby being applied to various fields.

### 2. Description of the Related Art

The term "superabsorbent polymer" used herein refers to a polymer having water absorbing potential of 100 times or more than an initial volume of the polymer due to an adsorption action of water using hydrophilic functional groups contained in the polymer.

In the superabsorbent polymer, polymer chains are crosslinked to some extent, such that the polymer itself absorbs water without being dissolved in water. In such a superabsorbent polymer, hydrophilic functional groups that cause a hydrogen-bond within a polymer chain structure are located at places very close to each other in the absence of moisture, and when water is introduced, hydrophilic functional groups absorb the water, and molecular structures swell and expand. Then, when an ambient temperature is increased to become a dry environment, the swollen molecular structures are contracted, such that the polymer releases the absorbed water, then returns to its original structure.

With the development of modern industry, standards for a hygiene level are rising. Meanwhile, when food is stored for a long period time, freshness of the food is reduced over time, and the food is unavoidably corrupted due to propagations of fungi and bacteria. Therefore, there have been many attempts to maintain the freshness of foods such as meats, fishes, vegetables, fruits and processed foods (hereinafter, collectively referred to as "foods") for a certain period of time and prevent contamination from various harmful external environments.

As an example of the attempts, research and development on antibacterial food packing materials that maintain freshness of food for a long period of time and prevent corruption caused by the bacteria and fungi have been actively conducted, and methods of antibacterial packing various products have been known in the art. Patent Document 1 (Korean Patent Registration No. 10-1780166) relates to an antibacterial food packing material containing metal nanoparticles and a method of manufacturing the same. In this manufacturing method, nanoparticles formed of metals such as silver, copper, and zinc brass having antibacterial properties are inserted into a plastic chip used as a packing material, and then subjected to a simple extrusion by a typical plastic molding method to manufacture an antibacterial film.

However, in a case of foods such as meats, fishes and fruits, a liquid is released from nutrients in these foods even when preserving the foods while preventing from coming into contact with an outside by the packing material, and the released liquid cannot be blocked by an antibacterial film. If the released liquid flows in the packed product, consumers may feel uncomfortable. Therefore, in recent years, superabsorbent polymers have been used in the food packing materials to prevent the above problems.

Meanwhile, high-performance superabsorbent polymers are used in infant and nursing diapers, or feminine sanitary napkins to prevent a leakage of liquid therefrom.

In the growing process of crops, when using soil mixed with the superabsorbent polymer, the superabsorbent polymer absorbs an excessive amount of water at an initial stage, and when the surrounding environment dries, slowly releases the absorbed water, such that it is possible to continuously supply water which is essential to grow the crops.

Meanwhile, liquids released from the foods or living organisms in a limited space contain water and various nutrients. When these nutrients contact bacteria, growth of the bacteria is promoted to cause a contamination.

The contaminants generated as above cause a rapid secondary contamination in objects which is used by a user for the required purpose. Particularly, in a case of infant and nursing diapers, or feminine sanitary napkins, pathogens contaminated by a body temperature of the human body are rapidly propagated. Thus, in spite of a short wearing time, various skin diseases may occur, and cause other diseases due to the propagation of bacteria in genitalia.

Attempts have been made to impart such a superabsorbent polymer with an antibacterial power using a variety of methods.

In Non-Patent Document 1, an antibacterial superabsorbent polymer is manufactured by adding a hydroethanolic solution of cetyltrimethylammonium bromide to an initial copolymer, and it was confirmed that the cetyltrimethylammonium bromide was uniformly dispersed into the superabsorbent polymer.

Although the polymer shows excellent antibacterial and sterilizing properties, there is a disadvantage in that absorbency of water varies depending on a concentration of cetyltrimethylammonium bromide and a ratio (%) thereof to water present in a solvent.

Patent Document 2 (Korean Patent Laid-Open Publication No. 10-2017-0009546) proposes a method of manufacturing an antibacterial superabsorbent resin by adding 0.2 to 0.9 parts by weight of Cu₂O oxide as a copper antimicrobial agent. In this method, a hydrogel phase polymer is dried and pulverized, and water and a copper antimicrobial agent are mixed therewith then stirred, followed by drying and pulverization to prepare a superabsorbent resin.

In Non-Patent Document 2, Cu₂O oxide is prepared by a physical method such as heat or UV irradiation or an oxidation-reduction method after immersing a cellulose fiber into a CuSO₄ solution. By using the prepared oxide, it was confirmed that there is an antibacterial effect against a brewing yeast (Saccharomyces cerevisiae). This document proposes that the Cu₂O oxide formed on the superabsorbent polymer has a strong antibacterial action, and that such a growth inhibiting action of the bacteria greatly helps to inhibit an oxidation of pineapple or melon juice.

Non-Patent Document 3 discloses that organic chemically modified red clay is added to a superabsorbent polymer to impart antibacterial properties. In this document, the modified red clay is dispersed in water, then mixed with a monomer of the superabsorbent polymer to prepare a superabsorbent polymer having the red clay dispersed therein. The superabsorbent polymer manufactured as described above allows various metal ions in the red clay to exhibit antibacterial and sterilizing effects while being gradually released. However, by using the red clay in which water is added to a monomer of the superabsorbent polymer, the number of manufacturing processes is further increased. In addition, since the synthesized superabsorbent polymer itself contains a large amount of water, there is a disadvantage that water absorption capacity is greatly reduced.

A method of impregnating superabsorbent polymer powders with a solution of silver colloidal is also known in the art. However, due to the aggregation of silver nanoparticles and excessive absorption of water, the water absorption capacity of the superabsorbent polymer is reduced.

The above-mentioned methods of imparting an antibacterial or sterilizing component to the superabsorbent polymer are summarized as follows. First, there is a method of adding an antibacterial or sterilizing component to the superabsorbent polymer by using a solution of metal salts through reduction with heat or UV light irradiation or a chemical reductant. As another method, there is a method of impregnating a superabsorbent polymer with chemicals such as an antimicrobial or sterilizing agent. As another method, there is a method of mixing powdery antimicrobial agents prepared by pulverizing organic or inorganic antimicrobial agents with powdery superabsorbent polymers. At this time, in order to increase an adhesion between the organic or inorganic antimicrobial agent and the superabsorbent polymer in a powder state, chemicals containing water or an organic solvent is used.

However, because the above-mentioned various methods use chemicals that contain water during the manufacturing process, the superabsorbent polymer may absorb the antimicrobial or sterilizing agent along with the water contained therein, thereby inherent properties of the superabsorbent polymer may be lost.

Therefore, it is necessary to add an impurity separation process or a moisture removing process. As a result, substances largely responsible for environmental pollution are discharged during the manufacturing process, as well as, there are problems such as an addition of a new process for removing these substances, an increase in manufacturing costs, a decrease in a water absorption rate, and an increase in a weight of the superabsorbent polymer.

### [Patent Document]

(Patent Document 1) Korean Patent Registration No. 10-1780166)
(Patent Document 2) Korean Patent Laid-Open Publication No. 10-2017-0009546

### [Non-Patent Document]

(Non-Patent Document 1) Sina Shahi, Mohammad J Zohuriaan-Mehr and Hossein Omidian," Antibacterial super absorbing hydrogels with high saline-swelling properties without get blockage: Toward ideal super absorbents for hygienic applications" Journal Bioactive and Compatible Polymers, 32(2) (2017), 128-145.
(Non-Patent Document 2) Amparo Llorens, Elsa Lloret, Pierre Picouet, and Avelina Fernandez" Study of the antifungal potential of novel cellulose/copper composites as absorbers for fruit juices" International Journal of Food Microbiology 158(2012) 113-119
(Non-Patent Document 3) K. Kabiri, H. Omidian, M. J. Zohuriaan-Mehr, and S.Doroudiani," rabsorbent Hydrogel Composites and Nanocomposite: A Review" Polymer Composite 32 (2011) 277-289.

### SUMMARY OF THE INVENTION

It is an object of the present invention to provide a method of adding metal nanoparticles to a superabsorbent polymer, which are harmless to a human body while maintaining water absorption capacity of the superabsorbent polymer and have excellent antibacterial properties.

Another object of the present invention is to provide a method of more economically manufacturing a superabsorbent polymer absorber which does not reduce water absorption capacity of the superabsorbent polymer, while allowing the metal nanoparticles formed thereon to provide an antibacterial power which inhibits a propagation of bacteria in a state rich in nutrients, a reducing power which delays an oxidation of blood in a human body, and a releasing power of essential minerals for promoting a growth of crops.

To achieve the above objects, according to an aspect of the present invention, there is provided a method of manufacturing a superabsorbent polymer absorber containing metal nanoparticles deposited on a surface thereof, the method including: (a) preparing superabsorbent polymer particles in a dried state; and (b) depositing metal nanoparticles on surfaces of the superabsorbent polymer particles.

Herein, the step of depositing the metal nanoparticles on the surfaces of the superabsorbent polymer particles may be performed in a vacuum deposition bath by a vacuum deposition method.

Further, the vacuum deposition method may include: generating metal vapor particles while stirring the superabsorbent polymer in a vacuum deposition bath provided with a stirring bath containing the superabsorbent polymer and a metal deposition source, so as to allow metal nanoparticles to be directly adhered to the superabsorbent polymer particles.

The present invention is advantageous in that the metal nanoparticles are directly adhered to the superabsorbent polymer particles during the manufacturing process, such that separate dispersion process and dispersant are not required. In addition, since an additive such as a reductant necessary for a chemical process is not required, it is possible to manufacture a superabsorbent polymer absorber containing metal nanoparticles deposited on the surface thereof with almost no inflow route of impurities.

The present invention may be used to produce existing diapers, sanitary napkins, food packing pads, etc., using the superabsorbent polymer absorber deposited with metal nanoparticles. At this time, the superabsorbent polymer of the present invention may replace the entire conventional superabsorbent polymer, or may be mixed with the conventional superabsorbent polymer to produce a product in the conventional manufacturing process and system.

In the present invention, the metal nanoparticles adhered to the superabsorbent polymer particles have a fine particle diameter of 2 to 30 nm, thereby having a high antibacterial, sterilizing or reducing power, and exhibiting activity of releasing mineral ions. Functional metal nanoparticles may be added to the superabsorbent polymer in a desired concentration within a range of 100 to 50,000 ppm to prepare an absorber without mixing of any water. In addition, since a part of the metal nanoparticles may be mixed with the conventional superabsorbent polymer, or may replace the entire conventional superabsorbent polymer, it is possible to produce a product having a new function imparted to the existing product without any additional process.

### BRIEF DESCRIPTION OF THE DRAWINGS

The above and other objects, features and other advantages of the present invention will be more clearly understood from the following detailed description taken in conjunction with the accompanying drawings, in which:
FIG. 1 is a schematic view illustrating a method of manufacturing an antibacterial superabsorbent polymer of the present invention;
FIG. 2 are photographs illustrating the superabsorbent polymer (a), a superabsorbent polymer deposited with silver nanoparticles (b), a superabsorbent polymer deposited with zinc nanoparticles (c), and mixed powders of the superabsorbent polymer deposited with silver nanoparticles and the superabsorbent polymer deposited with zinc nanoparticles (d), respectively;
FIG. 3 is a transmission electron microscope photograph of silver (Ag) which is one of additive metals of the present invention;
FIG. 4 is a photograph illustrating cases of Zn/SAP (a), Ag/SAP (b), a case in which water is added to Ag/SAP (c), a case in which water is added to Zn/SAP (d), and a case in which water is added to Ag/SAP and Zn/SAP (e), respectively;
FIG. 5 is photographs illustrating results of an antibacterial test of the superabsorbent polymer deposited with the silver nanoparticles prepared according to the present invention performed on E. coli, wherein experiment results between a control group and an experiment group performed on Staphylococcus aureus (SA), Klebsiella pneumoniae (KP), and E. coli. (Escherichia coli) are illustrated in this order from a left;
FIG. 6 is photographs illustrating degrees of decay from the 5th to 10th days after packing a mackerel in a typical zipper packing material;
FIG. 7 is photographs illustrating degrees of decay from the 5th to 10th days after adding 0.1 g of a conventional superabsorbent polymer to the typical zipper packing material then packing a mackerel therein;
FIG. 8 is photographs illustrating degrees of decay from the 5th to 10th days after adding 0.1 g of a Zn/superabsorbent polymer of the present invention to the typical zipper packing material then packing a mackerel therein;
FIG. 9 is photographs illustrating degrees of decay from the 5th to 10th days after packing a pork in the typical zipper packing material;
FIG. 10 is photographs illustrating degrees of decay from the 5th to 10th days after adding 0.1 g of a conventional superabsorbent polymer to the typical zipper packing material then packing a pork therein;
FIG. 11 is photographs illustrating degrees of decay from the 5th to 10th days after adding 0.1 g of the Zn/superabsorbent polymer of the present invention to the typical zipper packing material and then packing a pork therein;
FIG. 12 is photographs illustrating degrees of decay up to 7th day after packing white rice in a typical zipper packing material;
FIG. 13 is photographs illustrating degrees of decay up to 7th day after adding 0.1 g of conventional superabsorbent polymer to the typical zipper packing film then packing white rice therein; and
FIG. 14 is photographs illustrating degrees of decay up to 12th day after adding 0.1 g of the Zn/superabsorbent polymer of the present invention to the typical zipper packing material then packing white rice therein.

### DETAILED DESCRIPTION OF THE INVENTION

The present invention provides a method of manufacturing a superabsorbent polymer absorber containing metal nanoparticles deposited on a surface thereof, the method including: (a) preparing superabsorbent polymer particles in a dried state; and (b) depositing metal nanoparticles on surfaces of the superabsorbent polymer particles.

Hereinafter, the present invention will be described in detail. Terms or words used in the specification and claims should not be construed as limited to a conventional or lexical meaning, and should be understood as appropriate notions by the inventor based on that he/she is able to define terms to describe his/her invention in the best way to be seen by others.

A superabsorbent polymer absorber containing the metal nanoparticles prepared by a manufacturing method of the present invention has new functions such as antibacterial, sterilization, reduction and prevention of mineral loss due to the added metal nanoparticles, in addition to natural roles of the superabsorbent polymer such as an absorption of moisture from a flow of water and a prevention of rapid loss of mineral nutrients. The superabsorbent polymer absorber containing such metal nanoparticles may be used alone or in combination with conventional superabsorbent polymers to produce various products. In addition, it is preferable that the superabsorbent polymer absorber of the present invention is used together with a separation membrane made of various nonwoven fabrics or the like for preventing the nanoparticles from escaping to an outside. Further, when using the superabsorbent polymer absorber of the present invention by variously mixing with conventional natural and artificial fiber materials, it is possible to prevent the propagation of various bacteria contained in the liquid while absorbing various nutrient-containing liquids discharged from animals and plants.

The present invention provides a method of forming metal nanoparticles having an antibacterial, sterilizing, or reducing power, as well as, functions such as a far infrared ray emission, or mineral releasing on the superabsorbent polymer used as an absorber. The present invention relates to a method of forming metal nanoparticles having antibacterial/sterilizing functions on the superabsorbent polymer, by a method in which water and other impurities are not contained at all without an additional process such as moisture removal and impurity removal for solving the problems entailed in the conventional chemical method.

In addition, the present invention also relates to a use of the superabsorbent polymer absorber deposited with metal nanoparticles prepared by such a physical method. Since the present invention relates to a method of manufacturing an antibacterial superabsorbent polymer, and metal nanoparticles are added while maintaining the superabsorbent function of the superabsorbent polymer, the metal nanoparticles are formed in a uniform particle diameter. The metal nanoparticles having various functions may increase antibacterial properties due to a high dispersibility. In addition, since metal nanoparticles are present in an initial passage for absorbing nutrients along with water on the surface of the superabsorbent polymer, the nutrients come into contact with metal nanoparticles from the initial absorption process, and thereby effectively providing high antibacterial effects even at a low concentration.

Further, since the method of the present invention is a physical vacuum process, there is no inflow of impurities, and the main causative substances that pollute the environment are not discharged. In addition, since the various metal nanoparticles may be formed simultaneously or stepwise and a water-free process is employed, the formed metal nanoparticles may be stored for a long period of time. Further, the metal nanoparticles are kept in a stable state, such that the antibacterial and sterilizing abilities may be exerted regardless of the preservation period.

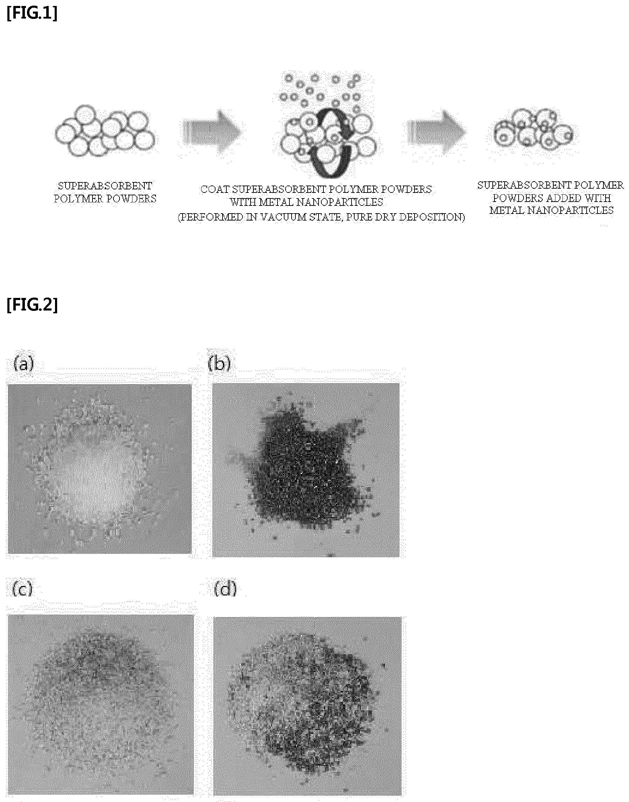

FIG. 1 is a schematic view illustrating the method of manufacturing a superabsorbent polymer absorber containing metal nanoparticles of the present invention.

It is preferable that the superabsorbent polymer containing metal nanoparticles deposited on the surface thereof is prepared by a method in which the superabsorbent polymer is used as a carrier in a vacuum deposition bath, and metal nanoparticles are vacuum deposited on the surface of the carrier.

Herein, the vacuum deposition will be described in more detail. The vacuum deposition may be performed by using an apparatus for vacuum depositing metal particles, which includes: a vacuum deposition bath; a stirring bath provided at a lower part of the vacuum deposition bath; a screw provided in the stirring bath to stir the carrier, that is, a superabsorbent polymer chip; and a metal deposition source provided at an upper part of the stirring bath in the vacuum deposition bath to generate metal vapor particles. A vacuum level in the vacuum deposition bath may be controlled to be in a range of 10⁻⁴ to 10⁻⁶ torr. When the vacuum level is less than 10⁻⁴ torr, vapor particles for forming nanoparticles are deposited thickly on the carriers near the metal deposition source for generating vapor particles in a low vacuum, whereas, as the carriers move away from the metal deposition source, a mean free movement distance of the vapor particles is shortened, such that vapor particles are not deposited on the carrier.

In the vacuum deposition, a rotation speed of the screw for stirring the superabsorbent polymer is preferably controlled to be in a range of 1 to 200 rpm, preferably 10 to 100 rpm. If the stirring speed is less than 1 rpm, there is a problem that the metal vapor particles are not uniformly adhered to the chip surface of the polymer since sufficient stirring is not performed. If the stirring speed exceeds 200 rpm, there is a problem that the stirred superabsorbent polymer particles are scattered and adhered to the metal deposition source, thereby causing electrical short-circuit.

The vacuum level in the vacuum deposition bath is controlled by including an inert gas. Herein, the inert gas may include argon (Ar), neon (Ne), N₂, O₂, CH₄ and the like.

In the step of generating vapor particles for forming nanoparticles using the above-described metal deposition source, physical vapor deposition methods may be used. As one example of the physical vapor deposition methods, thermal deposition such as a resistance heating method, plasma heating method, induction heating method and laser heating method, DC sputtering, DC-RF sputtering, laser sputtering, E-Beam evaporation and the like.

In the present invention, a deposition time may be set to be 10 minutes to 20 hours. By controlling the deposition time, the concentration of the metal particles with respect to the superabsorbent polymer may be controlled. That is, the deposition time may be controlled within the time range in order to maintain the concentration of the metal particles with respect to the superabsorbent polymer in a range of 100 to 3000 ppm depending on a concentration suitable for killing the bacteria or applications.

The vacuum deposition may control the growth of the nanoparticles by controlling the vacuum level in the deposition bath, the rotation speed of the screw, the deposition time, the deposition power, etc., such that the particle diameter and amount of the metal nanoparticles deposited on the superabsorbent polymer may be controlled.

Herein, any metal nanoparticle may be used so long as it is a nanoparticle of metal having antibacterial and sterilizing properties, and may include at least one selected from the group consisting of silicon (Si), copper (Cu), zinc (Zn), brass, aluminum (Al), beryllium (Be), magnesium (Mg), germanium (Ge), strontium (Sr), barium (Ba), yttrium (Y), titanium (Ti), zirconium (Zr), hafnium (Hf), vanadium (V), niobium (Nb), tantalum (Ta), lanthanum (La), silver (Ag), gold (Au), platinum (Pt), palladium (Pd) and an oxide thereof. Preferably, metals such as copper, silver, zinc, brass, and bronze are used.

The metal nanoparticles have a mean particle diameter of 2 to 30 nm, and preferably 2 to 10 nm.

When the nanoparticles are formed so as to have a particle diameter within the above range, aggregation between the nanoparticles is prevented, and the metal nanoparticles are well dispersed together with the absorption of water, such that metal nanoparticles having uniform properties may be achieved. That is, the nanoparticles prepared by the method of the present invention have a particle diameter of 2 to 30 nm, and preferably 2 to 10 nm, which is smaller than the particle diameter (50 to 100 nm) of the particles used in the metal vapor condensation method. Therefore, the surface area of the metal nanoparticles may be increased by a small amount. Further, there is no need for a separate process for removing a solvent or a dispersant by depositing the nanoparticles on the superabsorbent polymer without using a chemical dispersant, or a solvent.

Meanwhile, in a case of the prior art, a large amount of antimicrobial agent should be added because the nanoparticles are agglomerated together during the process due to insufficient dispersion. As a result, the price is not competitive and there is a limitation in an aspect of durability for the performance of the product using the superabsorbent polymer. On the other hand, the nanoparticles prepared by the method of the present invention are converted from raw materials to nanoparticles as they are, and addition or inflow of other foreign materials is prevented, such that there are advantages that the nanoparticles have a high purity, and excellent uniformity, without affecting properties of the superabsorbent polymer.

The superabsorbent polymer of the present invention is a polymer which is easily hydrogen-bonded to a water molecule (H₂O) such as CO, COOH, OH, NH, NH₂ or the like in a polymer chain, thereby having many functional groups capable of adsorbing water. In addition, the superabsorbent polymer refers to a polymer in which a certain amount of chains are cross-linked, and in the absence of moisture, the polymer chains retain completely twisted, whereas, when absorbing moisture, the polymer chains expand due to swelling, such that the polymer does not dissolve by crosslinking, and thereby having water absorbing potential to absorb 100 to 1000 times water based on an initial volume of the polymer.

As the superabsorbent polymer, any one may be used so long as it is a superabsorbent polymer in a form of particles conventionally used in the art without limitation thereof. As an example of the superabsorbent polymer, there is a polymer having a functional group such as CO, COO, OH, or NH which is a hydrophilic functional group capable of being hydrogen-bonded with water. Specifically, a hydrogel polymer, which is prepared by polymerizing a water-soluble ethylene unsaturated monomer or a monomer composition including a (meth)acrylate monomer and a polymerization initiator, may be used.

Preferably, the superabsorbent polymer particles used in the manufacturing method of the present invention have a mean particle diameter of 0.1 to 5 mm, and preferably 0.3 to 3 mm. If the metal nanoparticles have a particle diameter within the above range, after the metal nanoparticles are deposited on the surface of the polymer, and when the polymer absorbs the liquid to swell, the nanoparticles move into the polymer together with the liquid to exhibit an antibacterial effect or the like.

The present invention may use a commercially available 90 percent by weight ('wt.%') or more of dried superabsorbent polymer as it is, and may add a drying process if necessary.

The superabsorbent polymer absorber containing the metal nanoparticles deposited on the surface thereof prepared by the method of the present invention may be mixed with existing materials having functions of absorbing water or allowing water to pass, and the like, thus to perform various combinations of functions, for example, it can be utilized as a diaper, sanitary napkin, food packing absorber, blood absorbing band for medical applications and the like. In this case, due to the nanoparticles formed on the superabsorbent polymer, it is possible to perform an antibacterial or sterilizing function, enhancement of blood reducing action, far-infrared emission, moisture control in the target product or in the soil for crops, and sustained emission of minerals.

A water absorption or control product may be prepared by mixing the superabsorbent polymer absorber containing metal nanoparticles deposited on the surface thereof according to the present invention with materials having functions of absorbing water or allowing water to pass.

The water absorption or control product may be a diaper, a sanitary napkin, a food packing absorber, a blood absorbing band for medical applications, or a product configured to control the moisture in the soil for crops.

Hereinafter, the present invention will be described in detail with reference to examples. However, the examples according to the present invention may be modified into various other forms, and the scope of the present invention should not be construed as being limited to the embodiments described below. The examples of the present invention are provided to enable those skilled in the art to more fully understand the present invention.

### <Example 1> Preparation of superabsorbent polymer absorber containing metal nanoparticles deposited on the surface thereof

1-1. A disk type copper (Cu), bronze or silver (Ag) target having a diameter of 10 cm and a thickness of 1 cm was adhered to a DC sputtering cathode. 2-propenoic acid homopolymer sodium salt (sodium polyacrylate) containing 0-8 wt.% of water, which is produced by LG Chem, was used as a superabsorbent polymer. The polymer chip was introduced into a stirring bath in a vacuum deposition bath, then a door of the vacuum deposition bath was closed, followed by starting vacuum evacuation. The introduced polymer chip had a weight of 6.5 kg. After making a lower vacuum state of 1 x 10⁻² Torr inside of the stirring bath by using a rotary pump, the inside of the stirring bath was made into a high vacuum state of 1 x 10⁻⁵ Torr by using an oil diffusion pump.

In the high vacuum state, Ar gas was injected into the vacuum deposition bath at a flow rate of 50 to 150 seem, and the polymer chip was stirred at a speed of 60 rpm. At this time, injection of Ar gas is carried out to generate a plasma for deposition, and stirring of the superabsorbent polymer powders is carried out so that the particles to be deposited are maintained at a fine size without being coarse. When applying power to a DC power supply, a plasma was generated and deposition of metal particles was started. The deposition was carried out for 3 hours, and the concentration of the deposited metals was 0.1 wt.%, respectively.

FIG. 2 are photographs illustrating the superabsorbent polymer (a), a superabsorbent polymer deposited with silver nanoparticles (b), a superabsorbent polymer deposited with zinc nanoparticles (c), and mixed powders of the superabsorbent polymer deposited with silver nanoparticles and the superabsorbent polymer deposited with zinc nanoparticles (d), respectively. As illustrated in (a), (b), (c) and (d) of FIG. 2, it can be seen that the superabsorbent polymers and the superabsorbent polymers having the nanoparticles formed thereon have their initial shape and retain their outer appearance as they are. Through this, it can be confirmed that no water is absorbed since the work is performed in the vacuum deposition bath.

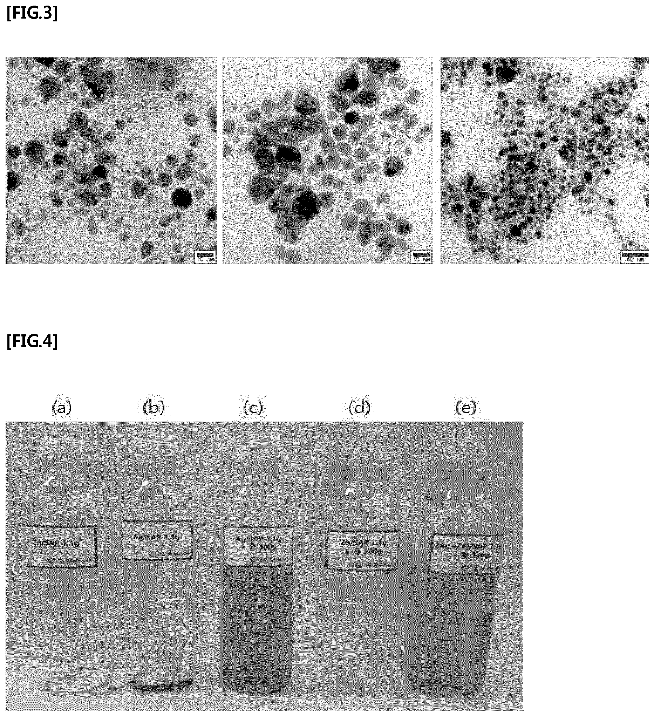

FIG. 3 is a transmission electron microscope photograph of silver nanoparticles. Herein, it can be seen that silver nanoparticles have a particle diameter of about 2 to 20 nm as a whole and have a mean particle diameter of about 5 to 7 nm.

### <Example 2> Experiment for absorbency

The manufacturing process of the present invention was performed in a vacuum state in which no water was used while having no connection with water. Therefore, an absorption rate of the superabsorbent polymer deposited with the metal nanoparticles of the present invention is similar to that of the initial superabsorbent polymer.
1.1 g of superabsorbent polymer (Zn/SAP) deposited with zinc nanoparticles, superabsorbent polymer (Ag/SAP) deposited with silver nanoparticles, and a mixture of Ag/SAP and Zn/SAP were put in typical beverage containers, respectively, then 300 ml of water was added thereto, and the appearances thereof were observed, respectively, and results thereof are shown in FIG. 4.

FIG. 4 illustrates results of observation in cases of Zn/SAP (a), Ag/SAP (b), a case in which water is added to Ag/SAP (c), a case in which water is added to Zn/SAP (d), and a case in which water is added to Ag/SAP and Zn/SAP (e), respectively. As a result of the observation experiment, it could be confirmed that after adding water, colors did not change regardless of the passage of time. That is, it can be seen that the superabsorbent polymer absorbed water and the metal nanoparticles formed on the surface of the superabsorbent polymer were uniformly dispersed into the superabsorbent polymer together with the water to be absorbed, as well as, the dispersed nanoparticles remained as they were without aggregation.

### <Example 3> Experiment for antibacterial property

A test for antibacterial activity of an absorber for a food packing material used for absorbing liquids from meats, fishes, and fruits by using the super absorbent polymer of the present invention was carried out. An antibacterial test was performed on the superabsorbent polymer having silver nanoparticles formed thereon by using an adhesion test method with film (JIS Z 2801), and results of the test were shown in the following tables. Bacteria used in the test were Staphylococcus aureus (SA), Klebsiella pneumoniae (KP), and Escherichia coli, and an aspect of changes for each strain are shown in Table 1, Table 2 and Table 3 below. No treatment was performed in the control group. The antibacterial activity values against bacteria were 6.3, 6.4 and 6.3 in cases of Staphylococcus aureus, Klebsiella pneumoniae, and Escherichia coli, respectively, in the experiment group, which showed much higher values than those of the typical antimicrobial agents.

**[Table 1]**

| Staphylococcus aureus | Control group | Experiment group |
|---|---|---|
| Initial number of bacteria | 1.8 x 10⁴ | 1.8 x 10⁴ |
| After 24 hours | 1.9 x 10⁴ | <0.63 |
| Antibacterial activity value | - | 6.3 |

**[Table 2]**

| Klebsiella pneumoniae | Control group | Experiment group |
|---|---|---|
| Initial number of bacteria | 1.1 x 10⁵ | 1.1 x 10⁵ |
| After 24 hours | 4.7 x 10⁷ | <20 |
| Antibacterial activity value | - | 6.4 |

**[Table 3]**

| Escherichia coli | Control group | Experiment group |
|---|---|---|
| Initial number of bacteria | 1.3 x 10⁴ | 1.4 x 10⁴ |
| After 24 hours | 1.3 x 10⁴ | <20 |
| antibacterial activity value | - | 6.3 |

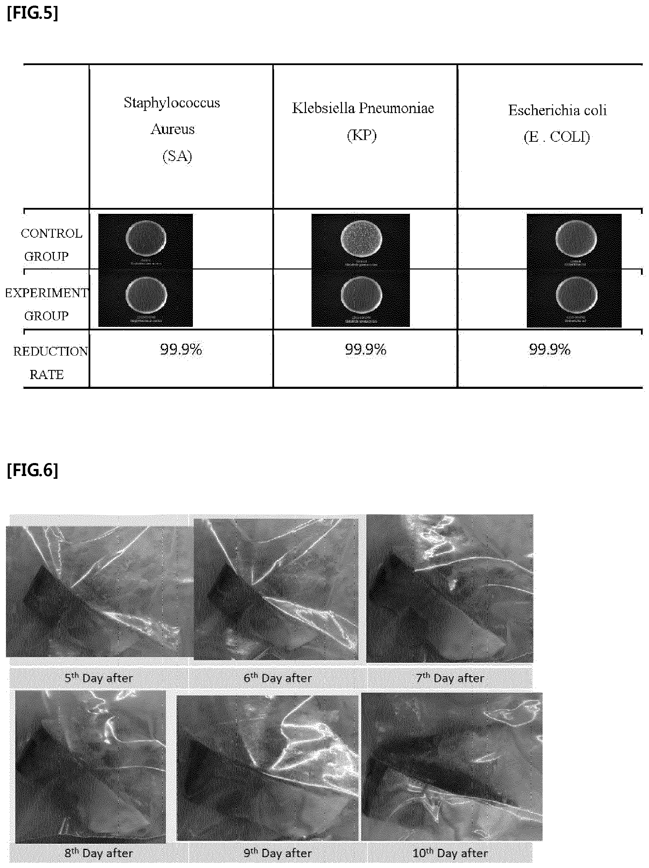

FIG. 5 illustrates photograph of the test results. As illustrated in the photographs, there are many white spots in the control group, which indicate the growth of the bacteria. However, in a case of the experiment group, there are almost no white spots, and the bacteria are killed and only a culture solution is visible. That is, it can be seen that the antibacterial power thereof is 99.9% or more.

### <Example 4> Experiment of food storage capability - seafood

A conventional superabsorbent polymer and the superabsorbent polymer having the zinc nanoparticles formed thereon of the present invention were put into a typical zipper packing material, and a mackerel was stored at room temperature up to the 10th day, then a degree of decay was determined, respectively.

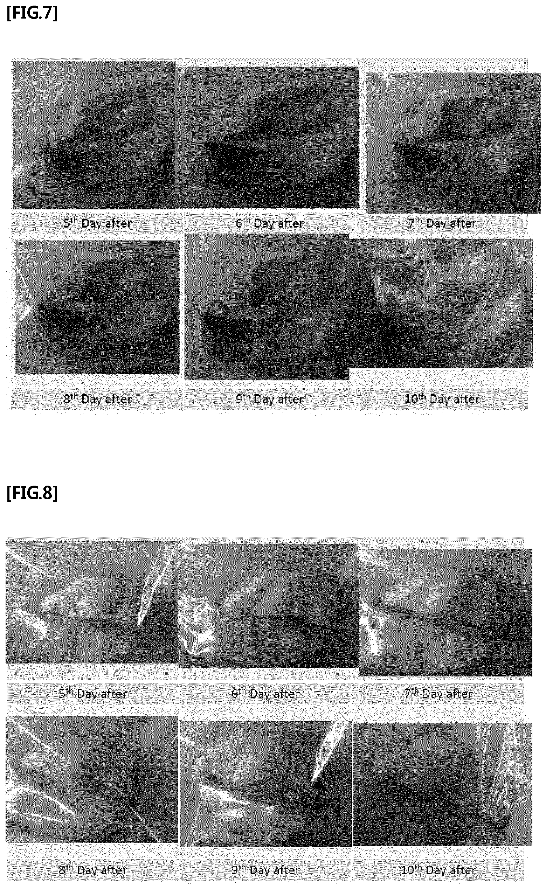

The experimental results are shown in FIGS. 6 to 8, which are photographs illustrating degrees of decay observed from the 5th to 10th days. FIG. 6 illustrates a case in which the mackerel is stored in a typical zipper packing material, FIG. 7 illustrates a case in which 0.1 g of a conventional superabsorbent polymer is put in the typical zipper packing material and the mackerel is stored therein, and FIG. 8 illustrates a case in which 0.1 g of Zn (1000 ppm)/SAP of the present invention is put in the typical zipper packing material and the mackerel is stored therein.

As illustrated in FIG. 6, in a case in which the superabsorbent polymer is not added to the typical zipper packing material, when seeing the photograph on the 5th day from the beginning, it can be seen that the reddish blood still remains, but a discharge liquid capable of grasping a propagation of bacteria is continuously created. In the case in which only 0.1 g of the conventional superabsorbent polymer is added to the typical zipper packing material in FIG. 7, as illustrated in the photograph, the degree of decay is very rapidly progressed. That is, in a case of pads using the superabsorbent polymer in order to give consumers a visually clean image by absorbing liquids from the meat in typical food packing, the superabsorbent polymer absorbs the liquids from the meats, thus it can be seen that the propagation of the bacteria is rapidly and intensively increased in the superabsorbent polymer. In addition, it can be seen that the form of mackerel is completely decomposed, and the degree of decay is very severe.

On the other hand, as illustrated in FIG. 8, in the case in which the Zn/SAP of the present invention is added to the typical zipper packing material, the degree of corruption is significantly reduced as compared with the case in which only the superabsorbent polymer is added. As evidence for this, it can be stated that the shape of the mackerel is preserved while still having reddish blood.

### <Example 5> Experiment of food storage capability - meats

A conventional superabsorbent polymer and the superabsorbent polymer having zinc nanoparticles formed thereon of the present invention were put into a typical zipper packing material, and a pork was stored at room temperature up to the 10th day, then a degree of decay was determined, respectively.

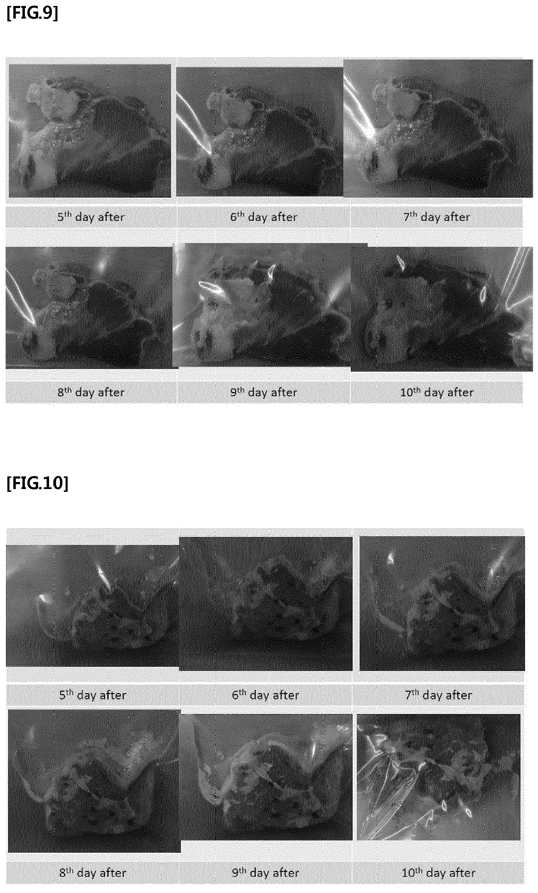

FIGS. 9 to 11 are photographs illustrating degrees of decay observed after 0.1 g of the superabsorbent polymer and the zinc (1000 ppm)/superabsorbent polymer prepared according to the present invention are added to the typical zipper packing material, and then a pork is put therein, followed by storing at room temperature, respectively.

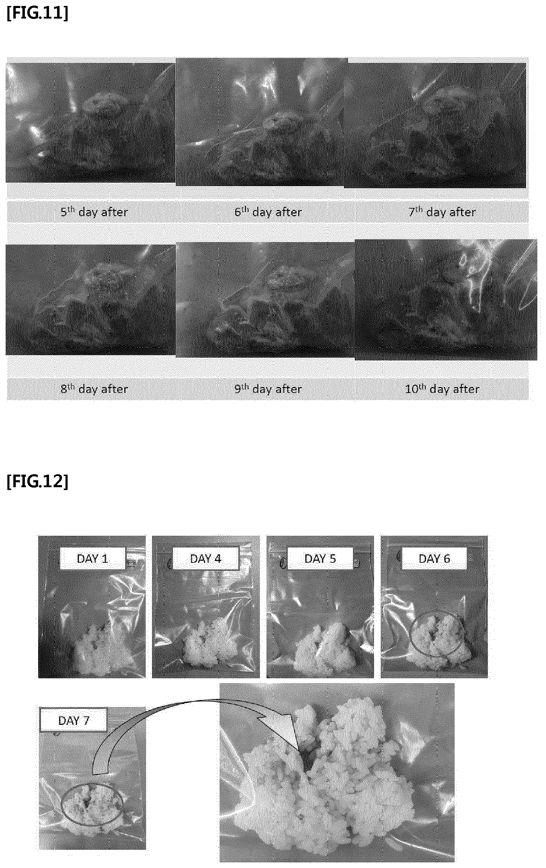

As illustrated in the photograph of FIG. 9, in a case in which the superabsorbent polymer is not added to the typical zipper packing material, when seeing the photograph on the 5th day from the beginning, it can be seen that the reddish blood still remains, but a discharge liquid capable of grasping a propagation of bacteria is continuously created. FIG. 10 is photographs illustrating degrees of decay observed from the 5th to 10th days after 0.1 g of the conventional superabsorbent polymer was added to the typical zipper packing material and the pork was stored therein. As illustrated in the photograph, it can be seen that the degree of decay is very rapidly progressed. That is, in a case of pads using the superabsorbent polymer in order to give consumers a visually clean image by absorbing liquids from the meat in typical food packing, the superabsorbent polymer absorbs the liquids from the meats, thus it may be seen that the propagation of the bacteria is rapidly increased in the superabsorbent polymer. In addition, it can be seen that the form of mackerel is completely decomposed, and the degree of decay is very severe. On the other hand, it can be seen that, in a case in which Zn (1000 ppm)/SAP is added to the typical zipper packing material in FIG. 11, the shape of pork is well maintained.

### <Example 6> Experiment of food storage capability - rice

A conventional superabsorbent polymer and the superabsorbent polymer having zinc nanoparticles formed thereon of the present invention were put into a typical zipper packing material, and rice was stored at room temperature up to 7th day, then a degree of decay was determined, respectively.

FIGS. 12 to 14 are photographs illustrating degrees of decay observed after 0.1 g of the superabsorbent polymer and the zinc (1000 ppm)/superabsorbent polymer prepared according to the present invention are added to the typical zipper packing material, and then rice is put therein, followed by storing at room temperature, respectively.

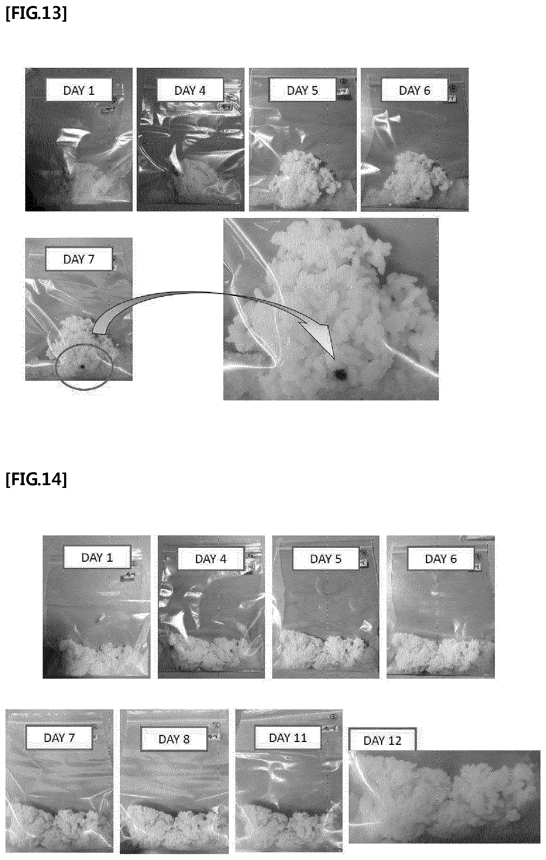

As illustrated in the photograph of FIG. 12, in a case in which the superabsorbent polymer is not added to the typical zipper packing material, when seeing the photograph on the 3rd day from the beginning, it can be seen that a color of the rice is changed from white to yellow. Through this, it can be seen that the propagation of bacteria continues. However, FIG. 13 is photographs illustrating degrees of decay observed up to 7th day after 0.1 g of the conventional superabsorbent polymer was added to the typical zipper packing material and rice was stored therein. As illustrated in the photograph, it can be seen that the color of the rice has been changed to yellow from the 4th day. Further, it can be seen that a lot of black molds are created on the 7th day.

On the other hand, it can be seen that, in a case in which the Zn/SAP of the present invention was added to the typical zipper packing material in FIG. 14, decay was not found until 7th day. As evidence for this, it can be stated that the shape of white rice was maintained. That is, it can be seen that, when adding the antibacterial superabsorbent polymer of the present invention, the degree of decay is completely different from that of the conventional superabsorbent polymer.

## Claims

1. A method of manufacturing a superabsorbent polymer absorber containing metal nanoparticles deposited on a surface thereof, the method comprising:
(a) preparing superabsorbent polymer particles in a dried state; and
(b) depositing metal nanoparticles on surfaces of the superabsorbent polymer particles.

2. The method according to claim 1, wherein the step of depositing the metal nanoparticles on the surfaces of the superabsorbent polymer particles is performed in a vacuum deposition bath by a vacuum deposition method.

3. The method according to claim 2, wherein the vacuum deposition method comprises: generating metal vapor particles while stirring the superabsorbent polymer in a vacuum deposition bath provided with a stirring bath containing the superabsorbent polymer and a metal deposition source, so as to allow metal nanoparticles to be directly adhered to the superabsorbent polymer particles.

4. The method according to claim 3, wherein the metal deposition source is selected from the group consisting of thermal deposition, electron beam deposition, DC sputtering, cathode arc deposition, DC magnetron sputtering, RF sputtering, ion beam sputtering, molecular beam epitaxy, arc discharge method and laser ablation.

5. The method according to claim 1, wherein the metal is selected from the group consisting of silicon (Si), copper (Cu), zinc (Zn), brass, aluminum (Al), beryllium (Be), magnesium (Mg), germanium (Ge), strontium (Sr), barium (Ba), yttrium (Y), titanium (Ti), zirconium (Zr), hafnium (Hf), vanadium (V), niobium (Nb), tantalum (Ta), lanthanum (La), silver (Ag), gold (Au), platinum (Pt), palladium (Pd) and an oxide thereof.

6. The method according to claim 1, wherein the superabsorbent polymer has CO, COOH, OH, NH, or NH₂ functional groups polymer chains, and the polymer chains are cross-linked.

7. The method according to claim 6, wherein the superabsorbent polymer has a water absorbing potential of 100 to 1000 times based on an initial volume thereof.

8. A water absorption or control product, comprising:
the superabsorbent polymer absorber prepared by the method according to claim 1; and
materials having functions of absorbing water or allowing water to pass, which are mixed with the superabsorbent polymer absorber.

9. The water absorption or control product according to claim 8, wherein the water absorption or control product is a diaper, a sanitary napkin, a food packing absorber, a blood absorbing band for medical applications, or a product configured to control the moisture in the soil for crops.
